Europäisches Patentamt

European Patent Office

Office européen des brevets

(11) Publication number: **0 284 419 B1**

## EUROPEAN PATENT SPECIFICATION

(45) Date of publication of patent specification: **11.05.94**　(51) Int. Cl.5: **A01H 1/02**

(21) Application number: **88302681.7**

(22) Date of filing: **25.03.88**

(54) Anther microspore-based selection process.

(30) Priority: **27.03.87 US 30987**

(43) Date of publication of application:
**28.09.88 Bulletin 88/39**

(45) Publication of the grant of the patent:
**11.05.94 Bulletin 94/19**

(84) Designated Contracting States:
**AT BE CH DE ES FR GB GR IT LI LU NL SE**

(56) References cited:
**EP-A- 0 154 204**
**GB-A- 2 139 466**
**US-A- 4 443 971**
**US-A- 4 528 773**

**RADIATION BOTANY, vol. 15, 1975, pages 1-16, Pergamon Press, GB; P.J. BOT-TINO:"The potential of genetic manipulation in plant cell cultures for plantbreeding"**

**I.K. VASIL: "CELL CULTURE AND SOMATIC CELL GENETICS OF PLANTS", vol. 1, 1984,pages 302-310, Academic Press Inc., London, GB; W.A. KELLER:"Anther culture of Brassica"**

**High frequency embrypgenesis through iso-**

lated microspore culture in Brassica napus
L. and B. carinata Braun. Chuong and Bever-
sdorf; Plant Science 39:219 (1985)

(73) Proprietor: **PIONEER HI-BRED INTERNATION-AL, INC.**
**Capital Square**
**400 Locust Square**
**Suite 700**
**Des Moines Iowa 50307(US)**

(72) Inventor: **Swanson, Eric B.**
**13 Malvern Crescent**
**Guelph Ontario N1H 6H8(CA)**
Inventor: **Beversdorf, Wallace**
**116 Applewood Crescent**
**Guelph Ontario N1H 6B6(CA)**
Inventor: **Coumans, Marc P.R.**
**RR2 Trafalgar Road**
**Georgetown Ontario L7G 4S5(CA)**

(74) Representative: **Hildyard, Edward Martin et al**
**Frank B. Dehn & Co.**
**Imperial House**
**15-19 Kingsway**
**London WC2B 6UZ (GB)**

Rank Xerox (UK) Business Services
(3.10/3.09/3.3.3)

## Description

Background of the Invention

The present invention relates to a process for in vitro exploitation of genetic variability in segregating gametic tissue, as well as variability induced by the application of mutagenic agents to gametic tissue, for the purpose of selecting mutant phenotypes. The present invention also relates to a selection procedure utilizing plant cells, including protoplasts, that are developed from microspores or from microspore-derived embryos.

The isolation of novel plant mutants by the application of selective growth conditions to cultured cells is well known. For example, Chaleff and Ray, "Herbicide-Resistant Mutants from Tobacco Cell Cultures," Science 223: 112-15 (1984), report isolating from cultured tobacco (Nicotiana tabacum) cells several mutants to the herbicides chlorsulfuron (Glean®) and sulfometuron methyl (Oust®), both products of E.I. du Pont de Nemours & Co. (Wilmington, Delaware, U.S.A.) The preparation of herbicide-tolerant tobacco, rice, corn, potato, oats, alfalfa, carrot and sugar cane plants is also the subject of U.S. patent No. 4,443,971, the contents of which are hereby incorporated by reference. The incorporated patent states, in particular, that a plant from among the foregoing types which is relatively tolerant of a herbicide selected from picloram, paraquat, 2,4-D, glyphosate, alachlor, atrazine and amitrole can be obtained by culturing tissue of a herbicide-sensitive parent plant in the presence of enough herbicide to kill at least 90% of the tissue initially present.

In these known selection systems, "tissue culturing" entails propagation of plant tissue, cells or protoplasts and regeneration of a whole plant therefrom. Crucial to the second step is the production of callus, a soft parenchymatous tissue comprised of large, thin-walled, rapidly dividing cells that can undergo differentiation to form other, more specialized tissues. In accordance with conventional selection systems, shoot formation from callus is induced, typically upon transferring callus tissue to a medium that contains an auxin like indole-3-acetic acid (IAA), a cytokinin or some other plant hormone. The shoots can then be grown into mature, whole plants which may express a trait, such as herbicide resistance, selected for at the cellular level. In this fashion, variability in the response of cultured tissue, cells or protoplasts to selective growth conditions is manifested in whole plants, which then are used as a source for the selected trait(s) in a breeding program.

The variation upon which the known selection systems draw is essentially somaclonal in nature, i.e., the variability is the result of spontaneous genetic changes that occur in somatic cells grown in tissue culture. The possible mechanisms which may underlie somaclonal variation -- changes at the individual-nucleotides level in a DNA molecule; the movement of transposable elements within chromosomes; more massive chromosomal abnormalities, including the loss or duplication of chromosome sections and the trading of segments between chromosomes -- have yet to be elucidated in detail. Nevertheless, it is known that a large, even predominant fraction of somaclonal variation arises during the period of tissue culture, and therefore does not result from some "unmasking" of variation present in the parent plant. Where mutagens are applied to these systems, the resulting variation is a mixture of somaclonal and mutagen-induced variability.

Tissue culture itself thus represents a severe perturbation to normal development and, hence, is responsible for much of the variability that is exploited by conventional in vitro selection systems. This presents a disadvantage because that variability is difficult, if not impossible, to control. Thus, the products of somaclonal variation are characteristically very poorly defined genetically. In particular, they frequently involve at least one decidedly undesirable trait, such as aneuploidy, polyploidy, genetic rearrangement (inversions, translocations) and other lethal or sub-lethal deficiencies, which detract from the utility of the plants regenerated therefrom. Keller in "Anther Culture of Brassica; Genetic Engineering Station; Ottawa Research Station" discloses a pathway leading to Brassica haploid regeneration, without any genetical selection, by means of microspore embryogenesis.

Summary of the Invention

Accordingly, it is an object of the present invention to provide a selection system for generating plant variants that does not rely primarily on somaclonal variation or where somaclonal variation may be one source of variation, the cells or protoplasts being derived directly from the gametic cell or resultant embryo.

It is also an object of the present invention to provide a process for exploiting genetic variation, in vitro, which process utilizes gametic cells, or tissue derived from embryos developed from gametic cells, as a tissue source.

It is another object of the present invention to provide a readily accessible source of desirable traits for rapid incorporation into important agronomic plants, such as rapeseed and other cruciferous crops.

It is a further object of the present invention to provide seed from which can be grown a plant that exhibits a desirable trait, such as tolerance to a herbicide.

In accomplishing the foregoing objects, there has been provided, in accordance with one aspect of the present invention, a process for producing plant variants, comprising the steps of (A) obtaining anther microspores in culture from a parent plant and separating said microspores from plant tissue and anther debris, (B) generating plant embryos from said microspores in the culture and (C) using the plant embryos to produce a whole plant, wherein said microspores, the plant embryos, or cells derived from said embryos are exposed to a selection agent that has a selective effect on the viability of microspores, embryos or plant cells, such that certain but not all of the exposed microspores, embryos or cells are viable, and the whole plant generated from the plant embryos is, unlike the parent plant, tolerant to the selection agent used. In another preferred embodiment, cells derived from the embryos in the form of protoplasts are exposed to the viability-affecting selection agent.

In accordance with another aspect of the present invention, plants have been provided that are the product of the above-described process, for example, where the agent is a sulfonylurea or an imidazolinone herbicide and the plant displays a tolerance of at least one of a sulfonylurea and an imidazolinone herbicide, which tolerance is not naturally-occurring. A rapeseed plant has been provided, for example, that displays a tolerance to chlorsulfuron, a sulfonylurea herbicide, that does not occur naturally in rapeseed.

Also provided, inter alia, is a plant, as well as seeds thereof, that displays a tolerance to an imidazolinone herbicide, which tolerance is independent of the presence in the plant of acetolactate synthase (ALS) enzyme that is tolerant of the herbicide. In a preferred embodiment, the plant does not display a tolerance of herbicides, other than the aforesaid imidiazolinone, that bind ALS enzyme.

Other objects, features and advantages of the present invention will become apparent from the following detailed description. It should be understood, however, that the detailed description and the specific examples, while indicating preferred embodiments of the invention, are given by way of illustration only, since various changes and modifications within the spirit and scope of the invention will become apparent to those skilled in the art from this detailed description.

Detailed Description of the Preferred Embodiments

The present invention involves the efficient production of a large number of microspores, the development therefrom of a large number of haploid plant embryos, and the direct regeneration from those embryos of whole plants which express a desired trait. The term "microspore" is used here to denote the smaller spore of a heterosporous plant (that is, a plant having both a megaspore and a microspore) which gives rise to a male gametophyte, the normal developmental product of which is the pollen grain. In accordance with the present invention, at least one selection agent, preferably in conjunction with a mutagenic agent, is employed to produce a subpopulation of microspores (or other plant cells obtained using tissue developed from microspores) that is characterized by the trait(s) desired for the regenerated plants.

It has been discovered that the microspore-based selection system of the present invention is sensitive to a broad range of chemicals, which can be used for selection in the system, with or without mutagenesis, at virtually any level of development of the embryos or plants. Perhaps more importantly, it has been found that the microspores used in the selection system of the present invention, since they represent the starting point in a direct path to whole plants via embryogenesis rather than somatic cloning, provide a tissue source that is more highly correlated (in terms of characterizing properties) with tissue in the whole-plant product than are tissue sources in known selection systems. Undifferentiated growth, a major disadvantage in conventional in vitro systems, is thus all but eliminated in the present invention. Moreover, the frequency with which desirable mutants are obtainable pursuant to the present invention-- typically, at a rate of 1 in about $10^4$ to $10^5$ microspore-derived embryos -- is readily accommodated in the context of in vitro microspore culture.

For purposes of this description, the phrase "selection agent" is used to designate a growth factor, either chemical or physical, that selectively influences the viability of cultured microspores to the effect that a subpopulation of those microspores is favored, after exposure to the agent, in subsequent embryogenesis. The phrase "mutagenic agent" refers to a growth factor that increases the level of variation in the properties of cultured microspores (or other plant cells in the system) which are exposed to the agent, but that does not work an unduly adverse effect on embryo development. Selection agents suitable for use in the present invention include cytotoxic chemicals such as the sulfonylurea herbicides, e.g., chlorsulfuron and sulfometuron methyl, and imidazolinone herbicides like AC 263,499 (Pursuit®; product of American Cyanamid). For purposes of the present invention, the sulfonylurea and imidazolinone herbicides are grouped together, despite their different chemistry, because both types of herbicides disrupt plant metabolism by acting on acetolactate synthase (ALS), also known as acetohydroxyl acid synthase (AHAS), the first enzyme in the pathway leading to production of leucine, isoleucine and valine in vivo.

Brassica plants are generally not known to display a natural tolerance to ALS-impairing herbicides, with the possible exception of a naturally-occurring resistance to the compound DPXY7881 (product of E. I. du Pont Co.). In the present description, the term "resistance" is used to denote the normal development of a plant after a usually effective dosage of a given selection agent has been applied. "Tolerance" pertains to a level of survival that is above a species norm but below complete resistance. Resistance to a given herbicide or other selection agent is thus the maximum tolerance at a particular level of the agent.

Exemplary of the other cytotoxic chemicals which can be used according to the present invention are paraquat, basagran, picloram, acifluorfen, 2,4-dichlorophenoxyacetic acid (2,4-D), glyphosate, alachlor, atrazine, cycloate, Basta (a product of Hoechst Co.), glufosinate-ammonium and amitrole.

As described in greater detail below, chemical hybridizing agents, such as those agents that have gametocidal activity ("gametocides"), like RH-531, RH-532 and RH-2956 (all products of Rohm-Haas Co., Nutley, New Jersey, U.S.A.), (2-chloroethyl)phosphonic acid (Ethrel) and cupferron, are suitable selection agents in the present invention. Similarly suitable are other substances that alter gametic development, such as the halogenated aliphatic acids $\alpha,\beta$-dichloro-isobutyrate (FW-450; product of Rohm-Haas Co.) and sodium 2,2-dichloropropionate (Dalapon®), antiauxins like maleic hydrazide and triiodobenzic acid, auxins like naphthaleneacetic acid and (at non-cytotoxic levels) 2,4-D, and gibberellins like GA4.

In another preferred embodiment, a host-specific toxin (HST) from a plant pathogen is employed in the present invention as a selection agent. Particularly preferred in this regard are the HSTs produced by saprophytic pathogens like Helminthosporium and Alternaria (see Table 1 below), which compounds can be used in the present invention to develop pathogen-resistant variants of tomato, citrus and other agronomic crops. In yet another preferred embodiment, the selection agent takes the form of an environmental factor, such as high and low temperatures, culture-medium salinity or pH, which can influence in vitro development and for which tolerance is desired.

## Table 1

### Host-Specific Toxins of _Alternaria_[1]

| Pathotype | Disease | Host | Toxin |
|-----------|---------|------|-------|
| Apple pathotype (_A. mali_) | Alternaria blotch of apple | Apple, pear | AM-toxin |
| Citrus pathotype (_A. citri_) | Brown spot of citrus | Citrus | AC-toxin |
| Japanese pear pathotype (_A. kikuchiana_) | Black spot of Japanese pear | Japanese pear | AK-toxin |
| Strawberry pathotype | Alternaria black spot of strawberry | Strawberry, Japanese pear | AF-toxin |
| Tobacco pathotype (_A. longipes_) | Brown spot of tobacco | Tobacco | AT-toxin |
| Tomato pathotype (_A. alternata_ f. sp. _lyopersici_) | Stem canker of tomato | Tomato | AL-toxin |
| Brassica pathotype[2] (_A. brassicae_) | Black spot of rapeseed | Rapeseed | $C_{30}H_{51}N_{15}O_7$ |

[1] Unless otherwise indicated, see Nishimura and Kohmoto, _Ann. Rev. Phytopathol._ 21: 87-116 (1983), the contents of which are hereby incorporated by reference.

[2] Bains & Twari, _Phytopathol._ 76(10): 1103 (1986), the contents of which are hereby incorporated by reference. See also Bains & Twari, _Physiol. Molec. Plant Pathol._ 30 (1987).

Also within the scope of the present invention are plants that have a pedigree comprised of a plant produced, as described above, using microspore-derived embroys. In the context of this description, the term "pedigree" denotes the lineage of a plant, e.g., in terms of the sexual crosses effected such that a combination of mutant genes, in heterozygous or homozygous condition, imparts a desired trait, such as herbicide tolerance, to the plant.

Suitable mutagenic agents for use according to the present invention include chemical mutagens like ethylmethane sulfonate and N-ethyl-N-nitrosourea. Irradiation with ultraviolet (UV) light, x-rays or gamma rays can also serve as a mutagenic agent, although some physical mutagens (such as gamma irradiation) may be of reduced effectiveness in view of their severe effects on the development of embryos.

Microspores are preferably obtained by homogenizing whole flower buds at high speed, in a microblender of the type Micro S/S sold by Eberbach Co. (Ann Arbor, Michigan), which microblender contains cool (about 12°C) wash medium (hormone-free B5, according to Gamburg et al., _Exp. Cell Res._ 50: 151-58 (1968)). Filtering of the homogenate yields large numbers of microspores free of tissue and cellular debris. Between 700 and 1,000 embryos per bud can be obtained routinely by this approach, with comparable yields and quality of microspores as have been reported for anther culture. The latter can also be used in the present invention as a source for microspores, in accordance with the disclosures of Chuong and Beversdorf, _Plant Sci._ 39: 219-26 (1985), and Lichter, _Z. Pflanzenphysiol._ 103: 229 (1981), the respective contents of which are hereby incorporated by reference. Generally, anthers obtained from flower buds of plants grown from diploid seed are macerated in a washing solution of B5 or other known medium, and the resulting suspension is aseptically filtered to obtain microspores.

It is especially preferred that the resulting isolated microspores be incubated overnight, at about 30°C, in a modified Lichter medium (microspore medium), see Lichter, _Z. Pflanzenphysiol._ 105: 427-434 (1982), containing 13 wt.% sucrose but no potato extract or hormones. After incubation, the microspores are centrifuged and resuspended in fresh microspore medium before being plated, typically in conventional petri dishes. Initiation of successful microspore culture, characterized by an increase in microspore volume, usually occurs within 24 hours at around 30°C. After about 5 to 7 days, cell clusters with well-defined epidermal layers (proembryos) are observed in culture, followed by the observation of typically heart-shaped or torpedo-shaped embryos within approximately 12 to 14 days. It is particularly preferred that the microspore cultures are maintained in darkness during this period. It is also preferred that, after about two weeks in culture, the embryos are subjected to mild shaking, which has been found to improve quality (percentage of torpedo-shaped embryos), synchrony and speed of embryo development.

After some three to four weeks in culture, torpedo-shaped embryos are transferred to a basal solid B5 medium with no hormones and 2% sucrose. Plant regeneration will occur directly from approximately 5 to 20% of these embryos, with later embryo development occurring upon subsequent subcultures. Alternatively, plant regeneration can be accomplished directly by placing the embryos, radicle downwards through a filterpaper interface, into Pro Mix which has been dampened with water. This "nursery" arrangement is maintained aseptically, e.g., in 100 x 25-mm deep petri dishes; the final moisture level should be approximately 15 mls of water per petri dish. The dish is placed in the light at approximately 150 $\mu E \cdot m^{-2} \cdot s^{-1}$ at 20-25°C. This procedure has produced as high as 90% direct plant regeneration from the embryos in 7 to 10 days. When proper torpedo-staged embryos are used, the initial shoots appear from the apical meristem, as is desired. Accordingly, direct plant regeneration after embryo isolation, in accordance with the present invention, permits plant development to proceed to the greenhouse in as little as 4 to 5 weeks after isolation.

In accordance with the present invention, a microspore culture prepared as described above is preferably exposed to a selection agent and, optionally, to a mutagenic agent prior to the time that the embryos are transferred to a solid medium. Alternatively, the parent plant can be exposed to the mutagenic agent prior to production from parent-plant tissue of microspores which are subsequently treated with the selection agent.

The microspore-derived embryos can themselves be exposed to the selection agent, with or without a prior mutagenesis step, before a viable embryo is used to produce a whole plant. In accordance with a preferred embodiment of the present invention, either the intact embryos or cells obtained from embryonic tissue can be treated with the selection agent; in the latter instance, the cells are preferably embryo-derived protoplasts, i.e., cells from which the pectocellulose cell walls have been removed, either by mechanical means or by enzymatic digestion. More specifically, protoplasts can be isolated from microspore-derived embryonic tissue, according to the present invention, by mechanical methods, for example, by gently tearing tissue apart in liquid culture medium with dissecting needles. Yields are generally low in this approach, however, so cell-wall removal by an enzymatic process is preferred, unless all side effects of wall-degrading enzymes must be avoided.

As described by Eriksson, "Protoplast Isolation and Culture," in PLANT PROTOPLASTS 1-20 (CRC Press 1983), the contents of which are hereby incorporated by reference, the enzymatic isolation of protoplasts from plant embryos produced from microspores, following the present invention, can be performed in two different ways: the two-step (or sequential method) and the one-step method. In the two-step method, the tissue is first treated with a macerozyme or a pectinase which separates the cells by degrading the middle lamella. The cells thus freed are then treated with cellulase, which releases the protoplasts. In general, the cells are exposed to the different enzymes for shorter periods than those used for the one-step method. In the one-step method the tissue is subjected to a mixture of enzymes, including macerozyme and cellulase. This method generally results in higher yields from leaf tissues since both mesophyll and palisade cells release protoplasts. The one-step method, which is also less labor intensive, is therefore preferred.

As previously indicated, the protoplasts thus obtained can be treated, with an optional prior exposure of the protoplasts to a mutagenic agent, with the chosen selection agent; the resulting subpopulation of viable protoplasts can then be used to regenerate whole plants expressing the property selected for in vitro. When cultured in one of the many suitable media available to the art, for example, as disclosed in PLANT TISSUE CULTURE METHODS, No. 19876 (Nat'l Research Council, Canada 1982), the protoplasts typically acquire an oval shape indicative of cell wall formation, and viable protoplasts will show cyclosis. Exemplary protocols developed for regenerating whole plants from protoplasts produced in accordance with the present invention are detailed in the following literature citations, which are hereby incorporated by reference: Barsby et al., Plant Cell Reports 5: 101-03 (1986); Spangenberg et al., Physiol. Plant 66: 1-8 (1986); Chuong et al., ,Plant Cell Reports 4: 4-6 (1985); Glimelius, Physiol. Plant 61: 38-44 (1984) and Kohlenbach, Z. Pflanzenphysiol. 105: 131-42 (1982).

The present invention is further described below by reference to the following examples.

Example 1. Variant Produced From Microspore-Derived Embryonic Tissue.

Plant Maintenance

Brassica napus L. (cv. "Topas") plants were grown in Pro Mix-C (Plant Products, Toronto, Canada) in 8-inch fiber pots. A 16-hour photoperiod of at least 400 $\mu$E m$^{-2}$•s$^{-1}$ at 18°C was used with an 8-hour dark interval at 13°C. Fertilizer 20-10-20 (N:P:K) was applied with routine watering (3 to 4 times per week) once plants were past the three-leaf stage.

Microspore Isolation

Young flower buds (0.5-5.0 mm) from the upper racemes of the B. napus plants were surface sterilized for 15 minutes in 5% sodium hypochlorite and given three five-minute rinses in sterile distilled water. The buds were placed in a cool (refrigerated) microblender (Micro S/S Eberbach Co.) containing cool (12°C) hormone-free B5 wash medium (4ml for every 10 buds) with 13% sucrose and blended at high speed for 6 to 7 seconds. The slurries from these treatments were passed through two layers of Nitex (pore size 48 $\mu$m, product of B.SH. Thompson Co., Toronto) and collected in centrifuge tubes. The blender and Nitex were then rinsed with B5 wash medium; the filtrates were centrifuged at 350g for ten minutes and the supernatant discarded. The pellet was resuspended in B5 wash and recentrifuged for two additional washes. The resulting microspores were suspended in a microspore medium, as described above, and incubated overnight at 30°C. The microspores were then recentrifuged and resuspended in fresh microspore medium.

Exposure of Microspores to Selection Agent

The microspores were counted using a Fuchs Rosenthal Ultra Plane corpuscle counting chamber, and 2.5ml of the microspore containing solution were plated, at 75,000 microspores per ml, in 60 x 15-mm petri dishes (Falcon 3002). Between the second and fifth days after isolation (an overnight incubation in the microspore medium constituting the first day), when cell division is initiated, the microspores were exposed to the mutagen N-ethyl-N-nitrosourea (ENU) by the addition of ENU to the medium to a concentration of between about 10 and 20 $\mu$M.

The microspores were then centrifuged to remove the ENU and were plated into petri dishes containing fresh microspore medium with 2.5 parts per billion (ppb) chlorsulfuron (test) and without chlorsulfuron (control), respectively. After approximately 30 days post-isolation, an average of 500 normal embryos were observed per control plate, while only two embryos had developed, out of 18,000 embryos anticipated based on the control average, in those dishes containing chlorsulfuron.

Plant Regeneration from Embryonic Tissue

The two embryos from the test (chlorsulfuron-treated) group were transferred to hormone-free B5 medium containing 0.45% agarose (Type 1, Sigma) and 2% sucrose but no chlorsulfuron. One embryo died after transfer; the remaining, chlorsulfuron-tolerant embryo developed a shoot, tissue from which was placed on B5 medium containing 5 ppb chlorsulfuron. The cultured shoot tissue continued to grow, in the presence of chlorsulfuron, into plantlets (micropropagation) which were subsequently transferred to Pro Mix.

At the 5-leaf stage, during active root growth, some of the plantlets were removed from the Pro Mix; after their roots were washed, these plantlets were placed in 0.2% colchicine for 5 to 6 hours. Thereafter, the roots of the colchicine-treated plants were washed again and the plants repotted in Pro Mix. Some of the branches of the repotted plants ("regenerated plants") were fertile and normal in appearance, while plantlets grown out with no colchicine treatment were haploid and sterile.

Seed produced by self-pollination of the regenerated plants provided plants ("inbred plants") that could also produce progeny ("inbred progeny") by self-pollination. In addition, the inbred plants were used in reciprocal crosses (i.e, as male and female, respectively) with normal Topas plants to produce progeny ("reciprocals") that were tested, along with regenerated plants, inbred plants and inbred progeny, for tolerance to the selection agent chlorsulfuron. The results, described in greater detail below, indicate that the regenerated plants contained a genetic determinant for chlorsulfuron tolerance that was inherited and expressed like a single, semidominant Mendelian gene.

Example 2. Variant Produced From Cells (Protoplasts) Obtained From Microspore-Derived Embryos.

Embryos were developed from B. napus (Topas) microspores as described in Example 1, with the difference that the microspores were exposed to neither a mutagen nor a selection agent during culturing. At three weeks after microspore isolation, the microspore-derived embryos were transferred from liquid medium to solid B5 medium (without hormones) and, after two more weeks, to solid MS medium (pH 6) supplemented with the auxin 2,4-dichlorophenoxyacetic acid (0.01 mg/l) and kinetin (0.05 mg/l). In the presence of these hormones, the cultured embryos became enlarged, and formation of secondary embryos was observed.

Protoplasts were then isolated from the enlarged embryos via a protocol similar to that of Barsby et al. (1986), incorporated by reference above. The basic protocol entails cell wall digestion in an enzymatic solution of 1.0% cellulase R-10 and 0.1% Macerozyme R-10. After filtration the protoplasts are rinsed and collected by flotation. The protoplasts are suspended at approximately 100,000 protoplasts/ml in the protoplast culture medium and then placed in Quadrant plates, with the protoplast culture medium in contact with the reservoir medium, as described by Shepard et al., Science 208: 17 (1980). Modifications to this protoplast isolation method included the removal of casein hydrolysate from the protoplast medium, and the addition 0.75 ppb chlorsulfuron to the protoplast medium but not to the reservoir medium. One callus colony survived and was transferred to dilution medium with 2.5 ppb chlorsulfuron. By contrast, Topas protoplasts were killed by 0.35 ppb chlorsulfuron when the herbicide was added to both the reservoir medium and the protoplast medium.

Shoot regeneration was induced by transferring the surviving colony to MS medium containing 1% sucrose, 100 mg/l casein hydrolysate, 2 mg/l kinetin, 2 mg/l zeatin and 0.01 mg/l IAA ("regeneration medium"). After 14 days (25°C; 16-hour photoperiod of 100 $\mu E \cdot m^{-2} \cdot s^{-1}$), the resulting nodular colony was transferred to fresh regeneration medium, and a shoot developed. When shoot tissue was micropropagated as described in Example 1, the resulting plants were not haploid and did produce seed via self-pollination.

The plants produced using the protoplasts of microspore-derived embryos were tested for chlorsulfuron tolerance, as were the different plant groups described in Example 1. In control experiments, levels of chlorsulfuron as low as 0.5 g/hectare were observed to kill normal Topas plants sprayed with the herbicide. As shown in Table 2, by contrast, all plants produced according to the present invention were at least an order of magnitude more chlorsulfuron-tolerant.

Table 2
Relative Magnitude and Heritability of
Chlorsulfuron (Glean®) Tolerance in
Mutant B. napus (cv. Topas) Plants and Plant Tissues

| Genotype: Glean® level: | Proto-plasts (ppb) | Micro-spores (ppb) | Ex-plants[1] (ppb) | Whole Plants[2] (PPI) 6 g/h | (PE) g/h |
|---|---|---|---|---|---|
| Topas (Control) | 0.5 | 2.5 | 2.5 | D | 0.5 |
| Regenerate I[3] | NA | 25.0 | 15.0 | S | 6.0 |
| Inbred Progeny of Regenerant I | NA | 25.0 | 15.0 | S | 6.0 |
| Reciprocal A[4] | NA | 25.0 (1/2) | 10.0 | S | 3.0 |
| Reciprocal B[5] | NA | 25.0 (1/2) | 10.0 | S | 3.0 |
| Regenerate II[6] | 100.0 | 80.0 + | 20.0 | S | 25.0 |
| Inbred Progeny of Regenerate II | NA | NA | NA | S | NA |

NA = Not attempted.
+ indicates limit of microspore tolerance is above highest level tested.
1/2 indicates that 1/2 the population was killed by 2.5 ppB and the other half survived up to 25 ppB.

1 Micropropagated shoot explants maintained on medium containing chlorsulfuron.

2 PPI (pre-plant incorporated) and PE (post-emergent) applications of chlorsulfuron involved spraying of Promix (1 week prior to sowing for PPI) or plants (at 3-5 leaf stage for PE) through a spray chamber, with 0.15% Agral 90 as a surfactant. D = Seeds germinated but no true leaves emerged and all plants died. S = Seeds germinated and all plants developed and flowered. All Topas control plants died at 1 g/h of chlorsulfuron so applied.

3 Regenerant I = Plants derived from microspore selection.

4 Reciprocal A = Plants from cross (Regenerant I x Topas).

5 Reciprocal B = Plants from cross (Topas x Regenerant I).

6 Regenerant II = Plants derived from selection from haploid protoplasts.

The level of chlorsulfuron required to kill the microspores was very distinct. Wild-type Topas microspores never produced an embryo when incubated at or above 3 ppb chlorsulfuron. Similarily, wild-type Topas never survived 6 g/h of chlorsulfuron applied PPI; indeed, no wild-type Topas plants even managed to produce any leaves beyond the cotyledons at 1 ppB applied PPI. In contrast, both selected mutants survived these (and more severe) treatments.

Biochemical studies on these mutants indicated that the specific enzyme activity for ALS, as determined by methods outlined by Haughn and Somerville, Molec. Gen. Genet. 204: 430-34 (1986), of regenerate I was 7 to 10 times more tolerant to chlorsulfuron than wild-type Topas. The progeny of reciprocal crosses of regenerate I with Topas had more chlorsulfuron-tolerant ALS enzymes, although the tolerance was generally less than plants from selfed seed of regenerate I. The sensitivity of the ALS enzyme of regenerate II to chlorsulfuron was the same as the wild-type Topas.

The % ALS activity and specific ALS activity were compared between the microspore and protoplast mutants and wild-type Topas plants, respectively, using the methods outlined by Haughn and Somerville (1986) and Chaleff and Mauvais, Science 224: 1443-45 (1984). The results indicated that the microspore mutant has an altered enzyme and that reciprocal hybrids of the microspore mutant to Topas also contain a less chlorsulfuron-sensitive enzyme. No difference in enzyme activity could be detected in leaves of the protoplast selected mutant.

The data summarized in Table 2 indicate, among other things, that plants produced using the in vitro selection system of the present invention expressed a tolerance to the selection agent that does not occur naturally in the parent cultivar. Moreover, the tolerance is stable across meiotic replication, as occurs when microspores are produced from regenerated plants, and is heritable, e.g., in regenerate I as a nonrecessive (semidominant) trait coded for by a single, nuclear genetic determinant.

Example 3. Comparison of Herbicide-Tolerant Mutants Produced From Mutagenized Microspores Selected On Pursuit®, An Imidazolinone Herbicide

In accordance with the protocols described in Example 1, embryos were developed from B. napus - (Topas) microspores cultured in the presence of 40 ppB of the herbicide Pursuit® [AC263,499], an imidazolinone ⟨5-ethyl-2-(4-isopropyl-4-methyl-5-oxo-2-imidazolin-2-yl)nicotinic acid⟩ produced by American Cyanamid (Princeton, NJ). Among the plants produced via this microspore selection, two mutants -- PM-1 and PM-2 -- were characterized in terms of a differential resistance to Pursuit® and/or Glean® (see Table 3 below). It was also determined that both mutants were more tolerant to the imidazolinone herbicide Assert (product of American Cyanamid) than parental material.

## Table 3

### Relative Magnitude and Heritability of Herbicide Tolerance in Two B. napus (Topas) Mutants

Highest Herbicide Level Tolerated[a]

| Genotype | PURSUIT Microspores (PPB) | GLEAN Plants (g/h) | Plants (g/h) |
|---|---|---|---|
| Parent Plant (Topas) | 30 | 10 | 1 |
| PM-1 (self seed)[b] | 500* | 300 | 1 |
| PM-2 (self seed)[c] | 500* | 500* | 50* |
| PM-1 x Parent | – | 100 | 1 |
| Parent x PM-1 | – | 100 | 1 |
| Parent x PM-2 | – | 100* | 30* |
| PM-2 x Parent | – | 100* | 30* |

\* Highest level tested

[a] For rapeseed, weed control generally requires about 100 to 200 g/h of Pursuit® or 10-20 g/h of Glean®, although levels in either case may be adjusted for particular weeds.

[b] In greenhouse studies with the above-indicated materials, the growth rate of PM-1 plants exposed, via spraying, to up to 100 g/h of Pursuit® was similar to parent-controls not treated with the herbicide. Thus, the PM-1 plants were resistant at a level of 100 g/h pre-plant incorporated (PPI). Higher levels of the herbicide were associated with significant indications of stress and delayed growth, i.e., the PM-1 plants displayed tolerance to the higher levels.

[c] There was no visible difference between the parent-controls and PM-2 plants when the latter were sprayed with herbicide up to 300 g/h of Pursuit® or up to 25 g/h of Glean® when the herbicides were PPI or post-emergently (PE) applied, respectively. Higher levels were well-tolerated, with some chlorosis of the leaves and meristems. PM-2 also tolerated up to 500 g/h of the imidazolinone herbicide Scepter®, and was resistant to 300 g/h of the herbicide, applied PPI or PE.

Both mutants were initially haploid and required treatment with colchicine, as described in Example 1, for fertility-restoration. The resulting plants were apparently euploid, with the pollen mother cells having a chromosome number of 19.

With the restoration of fertility, it became possible to produce plants having a pedigree that includes a plant with the herbicide-tolerance characteristics of PM-1 and/or PM-2. One illustration of the host of possible combinations within the present invention is a hybrid that is homozygous for PM-1 (i.e., a plant with both parents containing the PM-1 determinant) and heterozygous for PM-2 (only one parent with the PM-2 determinant).

Based on the results enumerated in Table 3, both PM-1 and PM-2 appeared to be less tolerant of Pursuit® in the heterozygous condition, and tolerance levels were similar in the progeny of reciprocal crosses. Thus, the genetic mechanism for tolerance in the PM-1 and PM-2 variants, respectively, is not cytoplasmic; nor is it recessive or simple-dominant in nature.

Biochemical studies conducted according to Haughn and Somerville, loc. cit., indicated that PM-2, which tolerated both imidazolinone and chlorsulfuron herbicides better than the parent cultivar, synthesized an ALS enzyme that was Pursuit®- and Glean®-resistant. In contrast, PM-1 possessed seemingly unaltered ALS and, while being comparatively tolerant of Pursuit®, displayed only a slight tolerance to Glean® and to Scepter®, another imidazolinone herbicide ⟨2-(4,5-dihydro-4-methyl-4-(1-methylethyl)-5-oxo-1H-imidazol-2-yl)-3-quinolinecarboxylic acid⟩ produced by American Cyanamid.

Significantly, spontaneous variation among microspore-derived embryos produced in accordance with Example 1, but without exposure of microspores to a mutagenic agent, did not yield chlorsulfuron-resistant variants at a detectable frequency, i.e., no variant was observed among some 250,000 embryos. This illustrates the characteristically low level of "uncontrolled" variability in the selection system of the present invention. In contrast to the genetic stability of the microspore culture exploited in the present invention, differences attributable to aneuploidy, mixoploidy and polyploidy, as well as to cytoplasmic variation, have been documented between calli (and even within calli) of the sort used in conventional selection systems. See generally D'Amato, "Cytogenetics of Plant Cell and Tissue Cultures and Their Regenerates," in 3 CRITICAL REVIEWS IN PLANT SCIENCES 73 (CRC Press 1985).

The present invention can be employed to produce valuable variants of any plant that is regenerable from microspores, either directly or through anther culture. Among the crops currently regenerable from microspores are barley, wheat, corn, sunflower, datura, sweet pepper, alfalfa, tobacco, rice, mushmelon, petunia, triticale, rapeseed and other members of Brassica, potato, tomato, barley, millet, coconut, onion, coffee, soybeans, Aesculus, Populus, Hevea and Orchidaceae.

Moreover, it will be appreciated that plants produced according to the present invention can be used in a seed production program, where the microspore system described above is applied to improve a specific character by the development of transgressive segregants. Examples of this application of the present invention include the following:

(A) Where a single tolerance gene is insufficient, multiple independent genes can be combined by crossing, and the transgressive recombinants are selected through microspore culture of the F1 progeny. In general, a level of the selection agent would be used that prevents the survival of microspores of either mutant alone, but that permits the survival of mutants with combined tolerance.

(B) This example could be used alone or in combination with example A above. Where a mutant plant has undesirable linkages or is in a poor agronomic background, a hybrid can be produced with a superior agronomic plant. Undesirable recombinants could subsequently be eliminated by applying the selection agent to the microspore medium. Selection for agronomic performance among the surviving plants (the phenotypes of which are fixed after spontaneous or colchicine-induced doubling) would be effected by conventional breeding methods.

This system would be especially useful where the desired selectable trait is recessive or involves more than one gene, or where the genetic background alters the expressivity or penetrance of the trait. The present invention can, therefore, be used to isolate a population of rare recombinants and, thereby, substantially reduce the time and cost for development of similar recombinants via conventional breeding methods.

Accordingly, seed that is produced by use of a selected trait directly or after further development, as illustrated above, constitutes a preferred embodiment of the present invention.

Because the microspore is a gametic cell, the selection system of the present invention can be used, in analogous fashion as described above, to generate variants of gametophytic cells, tissues and organs, for example, with regard to differential gametocide tolerance. Variants specific to sexual processes, such as pollen production, can also be produced via the present invention. Any agent that affects the number or viability of male or female gametes can be used as selection agents in this context. Aside from gametocides like those mentioned above, there are hormones and other compounds, used in "chemical hybridization" to alter gametic development, that can be used as selection agents in the present invention. See McRae, "Advances in Chemical Hybridization," Plant Breeding Rev. 3: 169-191 (1985), the contents of which are hereby incorporated by reference.

In addition, because the microspore selection system of the present invention involves embryogenesis that closely follows normal development of a seed, it is possible with the present invention to provide selective conditions for agronomic factors affecting embryo, seed or seedling development, which were heretofore all but inaccessible by means of conventional in vitro systems. These factors include cold and chilling tolerance; disease tolerance; resistance to herbicides; yield and seedling vigor. Thus, microspore-derived embryos can be exposed, pursuant to the present invention, to chilling or freezing conditions, and the survivors selected for propagation purposes, as described above. By the same token, the present invention can be applied in selecting within or between genotypes to obtain plants that respond more favorably to beneficial soil bacteria or to the growth-promoting substances produced by at least some of these bacteria, as described in copending application EP-A-87311026.6, the contents of which are hereby incorporated by reference.

**Claims**

1. A process for producing plant variants, comprising the steps of (A) obtaining anther microspores in culture from a parent plant, and separating said microspores from plant tissue and anther debris (B) generating plant embryos from said microspores in said culture and (C) using said plant embryos to produce a whole plant, wherein
   (i) said anther microspores, said plant embryos, or cells derived from said embryos are exposed to a selection agent that has a selective effect on the viability of microspores, embryos or plant cells, such that certain but not all of the exposed microspores, embryos or cells are viable; and
   (ii) said whole plant displays a tolerance to said selection agent.

2. A process according to Claim 1, wherein said tolerance is heritable.

3. A process according to Claim 1 or 2, wherein microspores from said parent plant are exposed to said selection agent.

4. A process according to Claim 1 or 2, wherein said plant embryos are exposed to said selection agent.

5. A process according to Claim 1 or 2, wherein cells derived from said embryos are exposed to said selection agent, said cells being protoplasts used in step (C) to produce said whole plant.

6. A process according to any of Claims 1 to 5, wherein said whole plant is tolerant of said selection agent, but said parent plant is not.

7. A process according to any of Claims 1 to 6, wherein said microspores, said plant embryos or said cells derived from said embryos are exposed to a mutagenic agent prior to exposure to said selection agent.

8. A process according to Claim 7, wherein said mutagenic agent is selected from N-ethyl-N-nitrosourea, ethylmethane sulfonate, X-irradiation, gamma irradiation and ultraviolet irradiation.

9. A process according to any of Claims 1 to 8, further comprising the step, prior to step (A), of exposing said parent plant to a mutagenic agent.

10. A process according to any of Claims 1 to 9, wherein said selection agent is a cytotoxic chemical.

11. A process according to Claim 10, wherein said cytotoxic chemical is picloram, paraquat, 2,4-D, glyphospate, alachlor, cycloate, glufosinate-ammonium, basagran, acifluorfen, atrazine or amitrole.

12. A process according to Claim 10, wherein said cytotoxic chemical is an imidazolinone herbicide or a sulfonylurea herbicide.

13. A process according to Claim 12, wherein said herbicide is chlorsulfuron or sulfometuron methyl.

14. A process according to Claim 12, wherein said herbicide is an imidazolinone.

EP 0 284 419 B1

**15.** A process according to any of Claims 1 to 9, wherein said selection agent is a compound that affects gametic development in said parent plant, a host-specific toxin produced by a pathogen or said parent plant, or a plant growth-promoting substance produced by soil bacteria.

**16.** A process according to any of Claims 1 to 15, wherein said microspores are obtained by homogenising whole flower buds of said parent plant at high speed.

**17.** A process according to claim 16, wherein said microspores are separated from plant tissue and cellular debris by filtration.

**18.** A process according to any of Claims 1 to 14, wherein said plant is a Brassica plant, said selection agent is a sulfonylurea or imidazolinone herbicide, and said whole plant displays a tolerance for a sulfonylurea herbicide and/or an imidazolinone herbicide, which tolerance is not naturally occurring in Brassica.

**19.** A process according to Claim 12, wherein said herbicide is chlorsulfuron.

**20.** A process according to Claim 18, wherein said herbicide is an imidazolinone.

**21.** A process of Claim 18, 19 or 20, wherein said plant is a B.napus plant

**Patentansprüche**

**1.** Verfahren zur Erzeugung von Pflanzenvarianten, umfassend die Stufen: (A) Erhalten von Antherenmikrosporen in Kultur aus einer parentalen Pflanze und Abtrennen der Mikrosporen von Pflanzengewebe und Antherenbruchstücken, (B) Erzeugen von Pflanzenembryos aus den Mikrosporen in der Kultur und (C) Verwenden der Pflanzenembryos zur Bildung einer vollständigen Pflanze, wobei
(i) die Antherenmikrosporen, die Pflanzenembryos oder von den Embryos abgeleitete Zellen einem Selektionsmittel ausgesetzt werden, das eine selektive Wirkung auf die Lebensfähigkeit der Mikrosporen, Embryos oder Pflanzenzellen in der Weise ausübt, daß bestimmte, jedoch nicht alle der behandelten Mikrosporen, Embryos oder Zellen lebensfähig sind; und
(ii) die vollständige Pflanze Toleranz gegenüber dem Selektionsmittel zeigt.

**2.** Verfahren nach Anspruch 1, wobei die Toleranz vererbbar ist.

**3.** Verfahren nach Anspruch 1 oder 2, wobei die Mikrosporen aus der parentalen Pflanze dem Selektionsmittel ausgesetzt werden.

**4.** Verfahren nach Anspruch 1 oder 2, wobei die Pflanzenembryos dem Selektionsmittel ausgesetzt werden.

**5.** Verfahren nach Anspruch 1 oder 2, wobei die von den Embryos abgeleiteten Zellen dem Selektionsmittel ausgesetzt werden, wobei es sich bei den Zellen um Protoplasten handelt, die in Stufe (C) zur Bildung der vollständigen Pflanze verwendet werden.

**6.** Verfahren nach einem der Ansprüche 1 - 5, wobei die vollständige Pflanze Toleranz gegenüber dem Selektionsmittel zeigt, dies aber für die parentale Pflanze nicht der Fall ist.

**7.** Verfahren nach einem der Ansprüche 1 - 6, wobei die Mikrosporen, die Pflanzenembryos oder die von den Embryos abgeleiteten Zellen vor der Behandlung mit dem Selektionsmittel einem mutagenen Mittel ausgesetzt werden.

**8.** Verfahren nach Anspruch 7, wobei das mutagene Mittel unter N-Ethyl-N-nitrosoharnstoff, Ethylmethansulfonat, X-Bestrahlung, $\gamma$-Bestrahlung und UV-Bestrahlung ausgewählt wird.

**9.** Verfahren nach einem der Ansprüche 1 - 8, ferner umfassend eine vor der Stufe (A) durchgeführte Stufe, bei der die parentale Pflanze einem mutagenen Mittel ausgesetzt wird.

13

## EP 0 284 419 B1

**10.** Verfahren nach einem der Ansprüche 1 - 9, wobei es sich beim Selektionsmittel um eine zytotoxische Chemikalie handelt.

**11.** Verfahren nach Anspruch 10, wobei es sich bei der zytotoxischen Chemikalie um Picloram, Paraquat, 2,4-D-Glyphosphat, Alachlor, Cycloat, Glufosinat-ammonium, Basagran, Acifluorfen, Atrazin oder Amitrol handelt.

**12.** Verfahren nach Anspruch 10, wobei es sich bei der zytotoxischen Chemikalie um ein Imidazolinon-Herbizid oder ein Sulfonylharnstoff-Herbizid handelt.

**13.** Verfahren nach Anspruch 12, wobei es sich beim Herbizid um Chlorsulfuron oder Sulfometuronmethyl handelt.

**14.** Verfahren nach Anspruch 12, wobei es sich beim Herbizid um ein Imidazolinon handelt.

**15.** Verfahren nach einem der Ansprüche 1 - 9, wobei es sich beim Selektionsmittel um eine Verbindung, die die gametische Entwicklung in der parentalen Pflanze beeinflußt, um ein von einem Pathogen oder der parentalen Pflanze gebildetes wirtsspezifisches Toxin oder um eine von Bodenbakterien gebildete, das Pflanzenwachstum fördernde Substanz handelt.

**16.** Verfahren nach einem der Ansprüche 1 - 15, wobei die Mikrosporen durch Homogenisieren von ganzen Blütenknospen der parentalen Pflanze bei hoher Geschwindigkeit erhalten werden.

**17.** Verfahren nach Anspruch 16, wobei die Mikrosporen von Pflanzengewebe und zellulären Bruchstücken durch Filtration abgetrennt werden.

**18.** Verfahren nach einem der Ansprüche 1 - 14, wobei es sich bei der Pflanze um eine Brassica-Pflanze handelt, es sich beim Selektionsmittel um ein Sulfonylharnstoff- oder Imidazolinon-Herbizid handelt und die vollständige Pflanze Toleranz für ein Sulfonylharnstoff-Herbizid und/oder ein Imidazolinon-Herbizid zeigt, wobei diese Toleranz in Brassica nicht von Natur aus vorkommt.

**19.** Verfahren nach Anspruch 12, wobei es sich beim Herbizid um Chlorsulfuron handelt.

**20.** Verfahren nach Anspruch 18, wobei es sich beim Herbizid um ein Imidazolinon handelt.

**21.** Verfahren nach Anspruch 18, 19 oder 20, wobei es sich bei der Pflanze um eine B. napus-Pflanze handelt.

**Revendications**

**1.** Procédé de production de variantes végétales, comprenant les étapes consistant à (A) obtenir des androspores à partir d'une plante mère en culture, et à séparer lesdits microspores du tissu végétal et des débris d'anthères (B) à générer des embryons végétaux à partir desdits microspores au cours de ladite culture et (C) à utiliser lesdits embryons végétaux afin de produire une plante entière, dans lequel
(I) lesdits androspores, lesdits embryons végétaux, ou lesdites cellules issues desdits embryons sont exposés à un agent sélectif qui exerce une action sélective sur la viabilité des microspores, des embryons ou des cellules végétales de façon telle qu'un certain nombre mais non la totalité des microspores, des embryons ou des cellules exposés soit viable; et
(II) ladite plante entière présente une tolérance audit agent sélectif.

**2.** Procédé selon la revendication 1, dans lequel ladite tolérance est héréditaire.

**3.** Procédé selon la revendication 1 ou 2, dans lequel des microspores issus de ladite plante mère sont exposés audit agent sélectif.

**4.** Procédé selon la revendication 1 ou 2, dans lequel lesdits embryons végétaux sont exposés audit agent sélectif.

14

**5.** Procédé selon la revendication 1 ou 2, dans lequel des cellules dérivées desdits embryons sont exposées audit agent sélectif, lesdites cellules étant des protoplastes utilisées lors de l'étape (C) pour produire ladite plante entière.

**6.** Procédé selon l'une quelconque des revendications 1 à 5, dans lequel ladite plante entière est tolérante audit agent sélectif, mais ladite plante mère ne l'est pas.

**7.** Procédé selon l'une quelconque des revendications 1 à 6, dans lequel lesdits microspores, lesdits embryons végétaux ou lesdites cellules dérivées desdits embryons sont exposés à un agent mutagène préalablement à leur exposition à un agent sélectif.

**8.** Procédé selon la revendication 7, dans lequel ledit agent mutagène est choisi parmi la N-éthyl-N-nitrosourée, le sulfonate d'éthylméthane, l'irradiation aux rayons X, aux rayons gamma et aux rayons ultraviolets.

**9.** Procédé selon l'une quelconque des revendications 1 à 8, comprenant également une étape qui consiste à exposer ladite plante mère à un agent mutagène préalablement à l'étape A.

**10.** Procédé selon l'une quelconque des revendications 1 à 9, dans lequel ledit agent sélectif est un produit chimique cytotoxique.

**11.** Procédé selon la revendication 10, dans lequel ledit produit cytotoxique est le picloram, le paraquat, le 2,4-D glyphosphate, l'alachlor, le cycloate, le glufosinate-ammonium, le basagran, l'acifluorfen, l'atrazine ou l'amitrole.

**12.** Procédé selon la revendication 10, dans lequel ledit produit cytotoxique est un herbicide à base d'imidazolinone ou à base de sulfonylurée

**13.** Procédé selon la revendication 12, dans lequel ledit herbicide est le chlorsulfuron ou le sulfométuron méthyle.

**14.** Procédé selon la revendication 12, dans lequel ledit herbicide est une imidazolinone.

**15.** Procédé selon l'une quelconque des revendications 1 à 9, dans lequel ledit agent sélectif est un composé qui agit sur la gamétogénèse chez ladite plante mère, une toxine spécifique d'hôte produite par un agent pathogène ou par ladite plante mère, ou une substance favorisant la croissance végétale produite par des bactéries du sol.

**16.** Procédé selon l'une quelconque des revendications 1 à 15, dans lequel lesdits microspores sont obtenus par homogénéisation à grande vitesse de boutons floraux entiers issus de ladite plante mère.

**17.** Procédé selon la revendication 16, dans lequel lesdits microspores sont séparés du tissu végétal et des débris cellulaires par filtration.

**18.** Procédé selon l'une quelconque des revendications 1 à 14, dans lequel ladite plante est une plante appartenant au genre Brassica, ledit agent sélectif est un herbicide à base de sulfonylurée ou d'imidazolinone, et ladite plante entière présente une tolérance vis-à-vis d'un herbicide à base de sulfonylurée et/ou d'imidazolinone, laquelle tolérance n'existant pas à l'état naturel chez Brassica.

**19.** Procédé selon la revendication 12, dans lequel ledit herbicide est le chlorsulfuron.

**20.** Procédé selon la revendication 18, dans lequel ledit herbicide est une imidazolinone.

**21.** Procédé selon la revendication 18, 19 ou 20, dans lequel ladite plante est B. napus.